# EUROPEAN PATENT SPECIFICATION

(11) **EP 4 115 857 B1**
(45) Date of publication and mention of the grant of the patent: **01.05.2024**
(21) Application number: 21778887.6
(22) Date of filing: 02.03.2021
(51) Int. Cl.: A61F 13/15, A61F 13/511, A61F 13/53, A61F 13/537

(54) **PRODUCTION METHOD FOR ABSORBENT ARTICLE**
HERSTELLUNGSVERFAHREN FÜR SAUGFÄHIGEN ARTIKEL
PROCÉDÉ DE PRODUCTION D'UN ARTICLE ABSORBANT

(30) Priority: 31.03.2020 JP 2020062767
(43) Date of publication of application: 11.01.2023
(73) Proprietor: Unicharm Corporation, Shikokuchuo-shi, Ehime 799-0111 (JP)
(72) Inventor: MIYAMA, Takuya, Kanonji-shi, Kagawa 769-1602 (JP); UDA, Masashi, Kanonji-shi, Kagawa 769-1602 (JP)
(74) Representative: Dolleymores
(86) International application number: PCT/JP2021/007877
(87) International publication number: WO 2021/199845

(56) References cited:
- WO-A1-2017/195386
- WO-A1-2020/040164
- JP-A- 2016 034 341
- JP-A- 2016 220 987

## Description

### FIELD

The present invention relates to a method for manufacturing an absorbent article in which a top portion and a bottom portion are located on a top sheet that comes into contact with the skin of a wearer.

### BACKGROUND

Hitherto, in absorbent articles that absorb excrement such as urine or feces of wearers, an absorbent article in which a protruded and recessed structure portion is located on a top sheet that comes into contact with the skin of the wearer has been provided (for example, refer to Patent Literature 1). The top sheet of Patent Literature 1 has a large number of pleated portions that are parallel to each other, and is joined to a base sheet between the pleated portions. The portion of the top sheet joined to the base sheet constitutes the bottom portion, and an apex portion of the pleated portion constitutes the top portion. Each pleat portion extends in a front-rear direction and is close to an adjacent pleated portion in a width direction. The bottom portion of the top sheet is configured to take in highly viscous excrement such as soft feces. The highly viscous excrement is taken into the bottom portion, which makes it possible to separate the body and the highly viscous excrement.

### [CITATION LIST]

### [PATENT LITERATURE]

Patent Literature 1: Japanese Patent Application Publication No. 2002-165830

### SUMMARY

### [TECHNICAL PROBLEM]

However, the top sheet of the absorbent article of Patent Literature 1 has the following problems. The top portion of the top sheet is floated from the base sheet and is spaced apart from the base sheet. Therefore, there is a case where a bodily fluid is less likely to be transferred from soft feces on the top sheet toward a non-skin facing surface side (base sheet side). In a state in which the soft feces remain on the top sheet, the top portion collapses due to body pressure or the like to bring the soft feces to be in a state of being attached to the skin, which may increase the load on the skin.

Therefore, the present invention was achieved in order to solve the above-described problems, and an aspect of the present invention is to enhance the transferability of bodily fluid to a non-skin surface on a top sheet having a protruded and recessed structure portion. The present invention provides a method for manufacturing an absorbent article as defined by claim 1. Preferred but optional features are defined in the dependent claims.

A method for manufacturing an absorbent article according to the preferred embodiment is to manufacture an absorbent article having a front waist region, a rear waist region, and a crotch region that is positioned between the front waist region and the rear waist region, and having a front-rear direction that is directed from the front waist region toward the rear waist region, and a width direction that is orthogonal to the front-rear direction. A method for manufacturing an absorbent article comprises: a top sheet transport step of transporting a top sheet continuous body in which a top sheet that comes into contact with a wearer is continuous; a laminate forming step of overlaying an auxiliary sheet that comes into contact with a non-skin surface of the top sheet on the top sheet continuous body to form a sheet laminate; and a shaping step of forming a protruded and recessed structure portion having a top portion that protrudes toward a skin facing surface side, a bottom portion that protrudes toward a non-skin facing surface side, and a wall portion that connects the top portion and the bottom portion in an overlaid region in which the top sheet and the auxiliary sheet overlap each other in the sheet laminate. In the method for manufacturing an absorbent article, In the shaping step, the protruded and recessed structure portion is formed with spaces in the front-rear direction at a position where at least a part of the protruded and recessed structure portion corresponds to the rear waist region.

### BRIEF DESCRIPTION OF DRAWINGS

FIG. 1 is an unfolded plan view of an absorbent article.
FIG. 2 is a cross-sectional view taken along a line A-A shown in FIG. 1.
FIG. 3 is a perspective view schematically showing a protruded and recessed structure portion.
FIG. 4 is a diagram schematically showing an example of a method for manufacturing an absorbent article and an apparatus for manufacturing an absorbent article.

### DESCRIPTION

### (1) Outline

At least following matters will become clear with description of this specification and attached drawings.

An absorbent article has a front waist region, a rear waist region, and a crotch region that is positioned between the front waist region and the rear waist region, and having a front-rear direction that is directed from the front waist region toward the rear waist region, and a width direction that is orthogonal to the front-rear direction, comprises: an absorbent core that straddles the crotch region and extends toward at least any one of the front waist region and the rear waist region; and a top sheet that is positioned on a skin facing surface side with respect to the absorbent core and comes into contact with a wearer. In at least the rear waist region, a protruded and recessed structure portion that has a top portion that protrudes toward the skin facing surface side, a bottom portion that protrudes toward a non-skin facing surface side, and a wall portion that connects the top portion and the bottom portion is located. The protruded and recessed structure portion is integrally formed with the top sheet and an auxiliary sheet that comes into contact with a non-skin facing surface of the top sheet in an overlaid region in which the top sheet and the auxiliary sheet are arranged. According to the present aspect, since the protruded and recessed structure portion is located in the rear waist region, excrement such as soft feces can be retained in the bottom portion between the top portions. Further, the protruded and recessed structure portion is integrally formed with the top sheet and the auxiliary sheet. That is, the protruded and recessed structure portion of the top sheet overlaps the protruded and recessed structure portion of the auxiliary sheet and extends along the protruded and recessed structure portion of the auxiliary sheet. Therefore, the moisture in excrement on the top sheet is easily transferred to the auxiliary sheet. Therefore, the transfer of bodily fluid from the top sheet to the auxiliary sheet can be facilitated while introducing the bodily fluid toward the non-skin facing surface side.

According to a preferable aspect, a density of the bottom portion is higher than a density of the wall portion. According to the present aspect, the density of the bottom portion is higher than the density of the wall portion, and the bodily fluid discharged on the top sheet is easily introduced into the bottom portion. The transfer of bodily fluid from the top sheet to the auxiliary sheet can be facilitated while introducing the bodily fluid toward the non-skin facing surface side.

According to a preferable aspect, a density of the top portion is higher than the density of the wall portion. According to the present aspect, since the density of the top portion and the density of the bottom portion are higher than the density of the wall portion, the bodily fluid discharged on the top sheet is easily introduced into the top portion and the bottom portion. When the bodily fluid is introduced into the bottom portion, the bodily fluid can be transferred to the auxiliary sheet while introducing the bodily fluid into the side away from the body. This makes it possible to suppress the problem of the bodily fluid continuing to come into contact with the skin. Further, the bodily fluid introduced into the top portion and introduced from the top portion toward the auxiliary sheet is introduced into the space on the back side of the bulge of the top portion. When the bodily fluid is drawn into the space on the back side of the top portion, the bodily fluid is introduced into the side away from the body, and the problem of the bodily fluid continuing to come into contact with the skin can be suppressed.

According to a preferable aspect, the absorbent article further includes a non-overlaid region in which the top sheet is arranged and the auxiliary sheet is not arranged, and the protruded and recessed structure portion is also formed in the non-overlaid region arranged on a rear side with respect to the overlaid region. According to the present aspect, in the non-overlaid region, the auxiliary sheet is not arranged, and the protruded and recessed structure portion is not integrally formed with the auxiliary sheet and the top sheet. Therefore, in the non-overlaid region, the drawability of the bodily fluid toward the non-skin facing surface side becomes low compared with the overlaid region. The non-overlaid region is positioned on the rear side with respect to the overlaid region, and there is a possibility that rear leakage may occur when an excessively large amount of bodily fluid is drawn. However, the drawability of the bodily fluid to the non-skin facing surface side of the non-overlaid region is relatively low, and the rear leakage of the bodily fluid can be suppressed. On the other hand, the overlaid region is positioned on the crotch side with respect to the non-overlaid region, and therefore, even when a large amount of bodily fluid is drawn, rear leakage is less likely to occur. Therefore, according to the above-described configuration, when the non-overlaid region and the overlaid region are located, absorption performance can be secured while suppressing rear leakage.

According to a preferable aspect, a density of the auxiliary sheet is higher than a density of the top sheet. According to the present aspect, the density of the auxiliary sheet is relatively high, and the transfer of bodily fluid from the top sheet toward the auxiliary sheet can be facilitated.

According to a preferable aspect, the absorbent article further includes a core wrap that covers the absorbent core and comes into contact with a non-skin surface of the auxiliary sheet, and a density of the core wrap is higher than the density of the auxiliary sheet. According to the present aspect, the density of the core wrap is relatively high, and the transfer of bodily fluid from the top sheet toward the auxiliary sheet and the transfer of the bodily fluid from the auxiliary sheet toward the core wrap can be facilitated.

According to a preferable aspect, the absorbent core has at least a superabsorbent polymer, and a weight of the superabsorbent polymer is 60% or more and 100% or less with respect to a weight of the absorbent core. According to the present aspect, since the weight of the superabsorbent polymer in the absorbent core is relatively high, the absorbent core is likely to swell when the bodily fluid is absorbed, compared with a configuration in which the ratio of pulp in the absorbent core is low. When the absorbent core swells and the absorbent core bulges toward the auxiliary sheet, the bottom portion of the auxiliary sheet becomes closer to the absorbent core, or the absorbent core enters the recess of the back side of the top portion of the auxiliary sheet, and therefore the drawability of the bodily fluid toward the absorbent core can be enhanced.

According to a preferable aspect, the overlaid region has a first overlaid region in which the protruded and recessed structure portion is located, and a second overlaid region in which the protruded and recessed structure portion is not located, and the second overlaid region is arranged on a front side with respect to the first overlaid region. According to the present aspect, in the first overlaid region in which the protruded and recessed structure portion is located, excrement can be drawn from the front surface side toward the back surface side of the top sheet. The moisture in the drawn excrement is transferred to the non-skin facing surface side. In addition, components excluding moisture in the excrement remain on the top sheet and are diffused along the recessed portions and the like of the protruded and recessed structure portion. At this time, since the second overlaid region in which the protruded and recessed structure portion is not located is arranged on the front side with respect to the first overlaid region, and the protruded and recessed structure portion for diffusing soft feces are not located in the first overlaid region, the diffusion of excrement toward the crotch side can be suppressed.

According to a preferable aspect, an area of the first overlaid region is smaller than an area of the second overlaid region. According to the present aspect, since the area of the first overlaid region is relatively small, soft feces can be locally retained and the diffusion of soft feces can be suppressed. Therefore, the load on the skin caused by the attachment of soft feces to the skin can be reduced.

A method for manufacturing an absorbent article according to the present invention is to manufacture an absorbent article having a front waist region, a rear waist region, and a crotch region that is positioned between the front waist region and the rear waist region, and having a front-rear direction that is directed from the front waist region toward the rear waist region, and a width direction that is orthogonal to the front-rear direction. A method for manufacturing an absorbent article comprises: a top sheet transport step of transporting a top sheet continuous body in which a top sheet that comes into contact with a wearer is continuous; a laminate forming step of overlaying an auxiliary sheet that comes into contact with a non-skin surface of the top sheet on the top sheet continuous body to form a sheet laminate; and a shaping step of forming a protruded and recessed structure portion having a top portion that protrudes toward a skin facing surface side, a bottom portion that protrudes toward a non-skin facing surface side, and a wall portion that connects the top portion and the bottom portion in an overlaid region in which the top sheet and the auxiliary sheet overlap each other in the sheet laminate. In the method for manufacturing an absorbent article, In the shaping step, the protruded and recessed structure portion is formed with spaces in the front-rear direction at a position where at least a part of the protruded and recessed structure portion corresponds to the rear waist region. According to the present aspect, an absorbent article in which the protruded and recessed structure portion integrally formed with the top sheet and the auxiliary sheet is located in at least the rear waist region can be manufactured.

According to a preferable aspect, in the laminate forming step, the top sheet continuous body and the auxiliary sheet are joined by thermal welding or ultrasonic welding. According to the present aspect, compared with a configuration in which the top sheet continuous body and the auxiliary sheet are joined via a hot-melt adhesive, a decrease in the drawability of the bodily fluid due to the adhesive can be suppressed. Therefore, the top sheet and the auxiliary sheet can be integrally formed while suppressing a decrease in the drawability of the bodily fluid, and the transferability of the bodily fluid from the top sheet toward the auxiliary sheet can be enhanced. Further, in a state in which the top sheet continuous body and the auxiliary sheet are joined, each sheet can be transported toward the shaping step, and problems such as folding during the transport of each sheet can be suppressed.

According to a preferable aspect, the shaping step includes a first shaping step of allowing the sheet laminate to pass between a first shaping roll and a second shaping roll that face each other to shape a part of the protruded and recessed structure portion, and a second shaping step of allowing the sheet laminate to pass between the second shaping roll and a third shaping roll that face each other to shape the other part of the protruded and recessed structure portion after the first shaping step. According to the present aspect, since the first shaping step and the second shaping step are provided, and the shaping process is performed in a stepwise manner, the protruded and recessed structure portion is easily formed to a detailed part compared with a step of forming the protruded and recessed structure portion at one time. Further, since the first shaping step and the second shaping step are performed using the common second shaping roll, a positional deviation between a part of the protruded and recessed structure portion formed by the first shaping step and the other part of the protruded and recessed structure portion formed by the second shaping step can be suppressed.

According to a preferable aspect, in the first shaping step, the protruded and recessed structure portion is shaped with spaces in a first direction that is one of the front-rear direction and the width direction, and in the second shaping step, the protruded and recessed structure portion is shaped with spaces in a second direction that is the other of the front-rear direction and the width direction. According to the present aspect, since the protruded and recessed structure portion with spaces in the first direction and the protruded and recessed structure portion with spaces in the second direction can be formed in a stepwise manner, the protruded and recessed structure portion can be appropriately molded while preventing damage to the material.

### (2) Absorbent article

Next, an absorbent article 10 will be described with reference to the accompanying drawings. The absorbent article absorbs the bodily fluid of a wearer. A target wearer can be exemplified by an infant and an elderly person. The absorbent article may be a disposable diaper or an incontinence pad that absorbs urine, a sanitary napkin that absorbs menstrual blood, or a panty liner that absorbs the vaginal discharge. The disposable diaper may be an underpants-shaped diaper or a tape-type diaper. The absorbent article according to the description and drawings is a tape-type disposable diaper. Further, in the following description of the drawings, identical or similar portions are denoted by identical or similar reference signs. Here, the drawings are schematic views, and attention needs to be paid to the fact that the ratios between individual dimensions and the like are different from actual ones. Therefore, specific dimensions and the like need to be determined with reference to the following description. In addition, there may be a portion where the relationship or ratio between dimensions does not match between drawings.

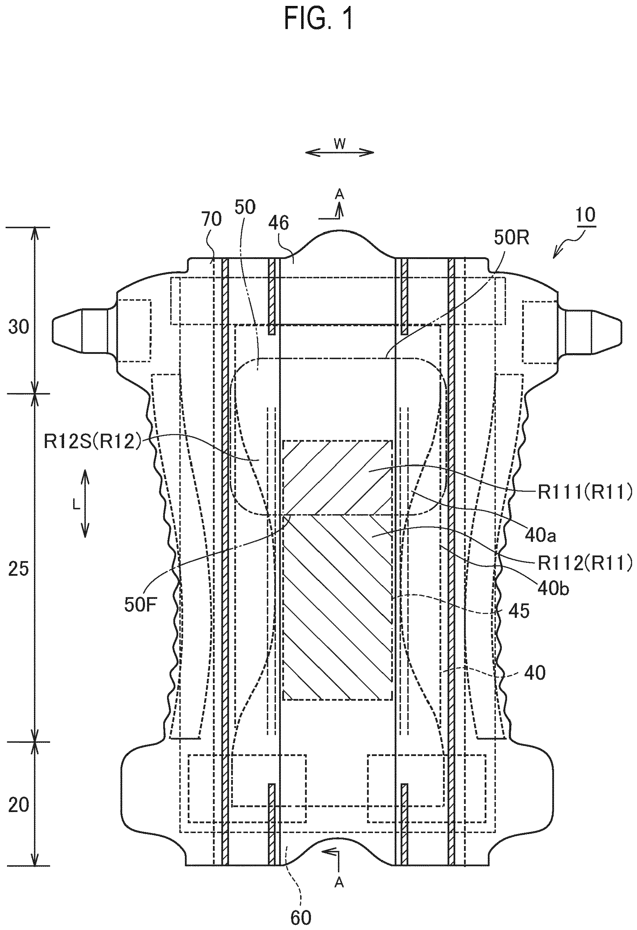

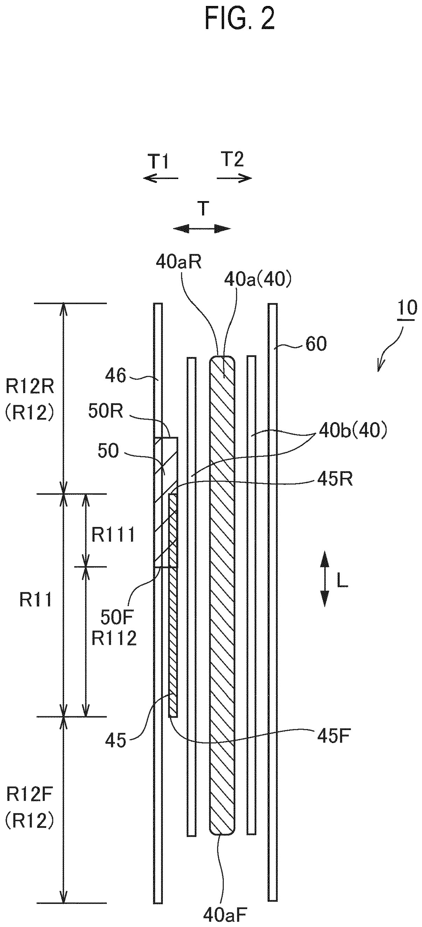

FIG. 1 is a schematic plan view of the absorbent article 10 as seen from a skin facing surface side T1 in an unfolded state. The schematic plan view shown in FIG. 1 shows a stretched state in which the absorbent article 10 is stretched to a state in which no wrinkles are formed. Fig. 2 is a schematic cross-sectional view taken along a line A-A shown in Fig. 1. In the schematic cross-sectional view, for convenience of description, each member may be spaced apart in the thickness direction T. However, in an actual product, each member is in contact with each other in the thickness direction T. The absorbent article 10 includes a front waist region 20, a crotch region 25, and a rear waist region 30. The front waist region 20 is a portion that comes into contact with the front waist portion (abdomen portion) of a wearer. Further, the rear waist region 30 is a portion that comes into contact with the rear waist portion (back portion) of the wearer. The crotch region 25 is positioned between the front waist region 20 and the rear waist region 30. In the absorbent article 10, it should be noted that a direction from the front waist region 20 toward the rear waist region 30 will be referred to as a front-rear direction L, a direction orthogonal to the front-rear direction L will be referred to as a width direction W, and a direction that extends to the skin facing surface side T1 and a non-skin facing surface side T2 of the wearer will be referred to as a thickness direction T.

The absorbent article 10 has an absorbent body 40. The absorbent body 40 may include an absorbent core 40a and a core wrap 40b. The absorbent core 40a includes an absorbent material such as pulp and a superabsorbent polymer. The absorbent core 40a straddles the crotch region 25 and extends toward at least any one of the front waist region 20 and the rear waist region 30. The absorbent core 40a of the absorbent article 10 is arranged to straddle the front waist region 20, the crotch region 25, and the rear waist region 30. In at least the rear waist region 30, a protruded and recessed structure portion 50 is located. The protruded and recessed structure portion 50 will be described in detail later. The core wrap 40b covers the absorbent core 40a. At least a part of the core wrap 40b comes into contact with the non-skin surface of an auxiliary sheet 45 described later.

The absorbent article 10 has a top sheet 46. The top sheet 46 is positioned on the skin facing surface side T1 with respect to the absorbent core 40a and comes into contact with the wearer. The top sheet 46 has liquid permeability. The top sheet 46 constitutes a skin contact surface of the absorbent article 10. The top sheet 46 is arranged to straddle the front waist region 20, the crotch region 25, and the rear waist region 30. The top sheet 46 has fibers, and specifically, can be formed of a nonwoven fabric. On the non-skin facing surface side T2 of the absorbent body 40, a liquid-impermeable back sheet 60 is provided. On the non-skin facing surface side T2 of the back sheet 60, an exterior sheet (not shown) formed of a nonwoven fabric may be arranged.

The absorbent article 10 has the auxiliary sheet 45 that comes into contact with the non-skin facing surface side T2 of the top sheet 46. The auxiliary sheet 45 is arranged between the top sheet 46 and the absorbent body 40. The auxiliary sheet 45 is arranged between the top sheet 46 and the core wrap 40b. The area of the auxiliary sheet 45 may be smaller than the area of the top sheet 46. More specifically, the length of the auxiliary sheet 45 in front-rear direction L may be shorter than the length of the top sheet 46 in the front-rear direction L and may be shorter than the length of the absorbent core 40a in the front-rear direction L. The length of the auxiliary sheet 45 in the width direction W may be shorter than the length of the top sheet 46 in the width direction W and may be shorter than the length of the absorbent core 40a in the width direction W. An overlaid region R11 in which the top sheet 46 and the auxiliary sheet 45 are overlaid on each other, and a non-overlaid region R12 in which the top sheet 46 is arranged and the auxiliary sheet 45 is not arranged (the top sheet 46 and the auxiliary sheet 45 are not overlaid on each other) may be located. The non-overlaid region R12 may include a rear non-overlaid region R12R that is positioned on the rear side with respect to the overlaid region R11, a front non-overlaid region R12F that is positioned on the front side with respect to the overlaid region R11, and a side non-overlaid region R12S that is positioned on the outer side in the width direction W with respect to the overlaid region R11. The rear non-overlaid region R12R is a region from a rear end edge 45R of the auxiliary sheet 45 to a rear end edge 46R of the top sheet 46 (rear end edges of the absorbent article). The front non-overlaid region R12F is a region from a front end edge 45F of the auxiliary sheet 45 to a front end edge 46F of the top sheet 46 (the front end edges of the absorbent article). The side non-overlaid region R12S is a region between an extension line that passes through an outer side edge of the auxiliary sheet 45 and extends in the front-rear direction L, and an outer side edge of the top sheet 46.

### (3) Configuration of Protruded and Recessed Structure Portion

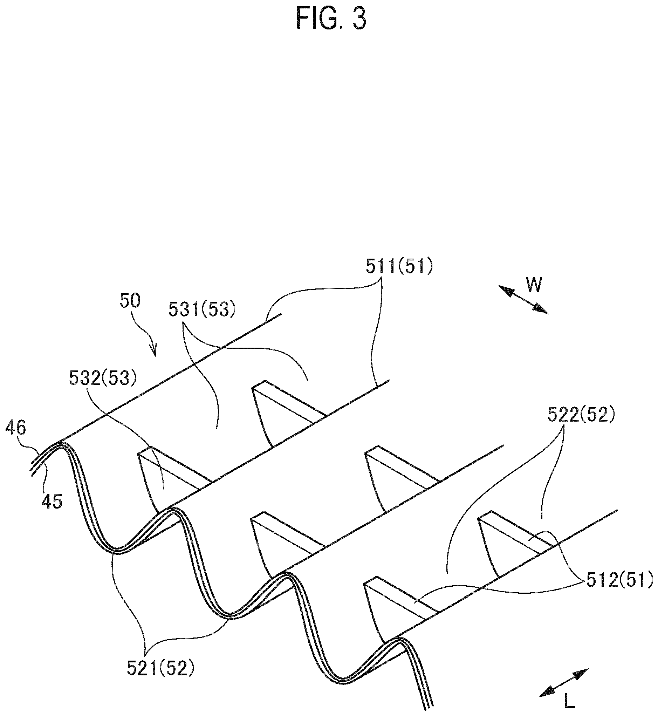

Next, the configuration of the protruded and recessed structure portion 50 will be described in detail. FIG. 3 is an enlarged plan view of the protruded and recessed structure portion 50. The protruded and recessed structure portion 50 includes top portions 51 that protrude toward the skin facing surface side T1, bottom portions 52 that protrude toward the non-skin facing surface side T2, and wall portions 53 that connect the top portions 51 and the bottom portions 52. In FIGS. 1 and 2, the detailed structure of the protruded and recessed structure portion 50 is omitted. The top portion 51 protrudes toward the skin facing surface side T1 with respect to the surrounding region. The bottom portion 52 protrudes toward the non-skin facing surface side T2 with respect to the surrounding region. The wall portion 53 is a portion that connects the top portion 51 and the bottom portion 52, and may extend in at least the thickness direction T. The top portion 51 is a portion including a point positioned on the most skin facing surface side T1, the bottom portion 52 is a portion including a point positioned on the most non-skin facing surface side T2, and the wall portion 53 is a portion between the top portion 51 and the bottom portion 52.

The protruded and recessed structure portion 50 of the absorbent article 10includes first top portions 511, first bottom portions 521, and first wall portions 531, each of which extends in the front-rear direction L, and second top portions 512, second bottom portions 522, and second wall portions 532, each of which extends in the width direction W. The first top portions 511, the first bottom portions 521, and the first wall portions 531 are arranged with spaces in the width direction W, and are arranged to be adjacent to each other in a cross section along the width direction W. Further, the second top portions 512, the second bottom portions 522, and the second wall portions 532 are arranged with spaces in the front-rear direction L, and are arranged to be adjacent to each other in a cross section along the front-rear direction L. As described above, in the aspect including the protruded and recessed structure portion 50 that extends in the width direction W and the protruded and recessed structure portion 50 that extends in the front-rear direction L, the diffusion of feces in the width direction W can be suppressed and the diffusion of feces in the front-rear direction L can also be suppressed. In addition, in an aspect having the protruded and recessed structure portion 50 that extends in the width direction W and the protruded and recessed structure portion 50 that extends in the front-rear direction L, the top portion and the bottom portion can be set in each of cross sections of the top portion and the bottom portion (that extend in the front-rear direction L) in a cross section along the width direction W, and the top portion and the bottom portion (that extend in the width direction W) in a cross section along the front-rear direction L. Further, the protruded and recessed structure portion 50 may have any form as long as the structure portion has a top portion, a bottom portion, and a wall portion, and the configuration is not limited to the present example. A well-known structure can be employed. For example, a protruded and recessed structure portion 50 described in Japanese Patent No. 6087462 can be employed.

The protruded and recessed structure portion 50 is integrally formed with the top sheet 46 and the auxiliary sheet 45 in the overlaid region R11 in which the top sheet 46 and the auxiliary sheet 45 are arranged. That is, the protruded and recessed structure portion 50 is formed on the skin facing surface (the surface that comes into contact with the skin of the wearer) of the absorbent article. It should be noted that in the aspect in which the protruded and recessed structure portion is integrally formed with the top sheet 46 and the auxiliary sheet 45, the protruded and recessed structure portion (for example, protruding portions) of the top sheet 46 and the protruded and recessed structure portion (for example, protruding portions) of the auxiliary sheet 45 are not separately formed, both are formed together, and the protruding portions of the auxiliary sheet 45 are arranged along the protruding portions of the top sheet 46. In a method for manufacturing an absorbent article described below, the protruded and recessed structure portion 50 subjected to the shaping process in a state in which the top sheet 46 and the auxiliary sheet 45 are overlaid on each other becomes a protruded and recessed structure portion 50 which is integrally formed with the top sheet 46 and the auxiliary sheet 45.

The protruded and recessed structure portion 50 is arranged in at least the rear waist region 30. Therefore, feces are mainly discharged onto the protruded and recessed structure portion 50. When feces are discharged onto the protruded and recessed structure portion 50, the feces are stored in the bottom portions 52 between the top portions 51. By drawing the feces into the protruded and recessed structure portion 50, discomfort caused by the feces continuing to come into contact with the skin of the wearer can be reduced. Further, the protruded and recessed structure portion 50 is integrally formed with the top sheet 46 and the auxiliary sheet 45. That is, the protruded and recessed structure portion 50 of the top sheet 46 overlaps the protruded and recessed structure portion 50 of the auxiliary sheet 45 and extends along the protruded and recessed structure portion 50 of the auxiliary sheet 45. Therefore, the moisture in excrement on the top sheet 46 is easily transferred to the auxiliary sheet 45. Therefore, the transfer of the bodily fluid from the top sheet 46 to the auxiliary sheet 45 can be facilitated while introducing the bodily fluid toward the non-skin facing surface side T2.

The density of the bottom portion 52 may be higher than the density of the wall portion 53. It is sufficient that the density of at least a part of the bottom portion 52 is higher than the density of the wall portion 53, and the bottom portion 52 may have a portion having the same density as the wall portion 53. Since the density of the bottom portion 52 is higher than the density of the wall portion 53, the bodily fluid discharged onto the top sheet 46 is easily introduced into the bottom portion 52. The transfer of the bodily fluid from the top sheet 46 to the auxiliary sheet 45 can be facilitated while introducing the bodily fluid toward the non-skin facing surface side. It should be noted that, a portion having a high density and a portion having a low density can be determined by the following method. The density can be determined by enlarging a target portion of the sheet (for example, a size of 1 mm × 1 mm) by a digital microscope, an electron microscope, or the like to approximately 100 times, and comparing the number of fibers present on the sheet surface in a unit area. Preferably, 30 positions are checked in each of the regions to be measured, and, from the tendency of the number comparison, a target portion where the number of fibers is likely to be large is defined as a region where the fiber density is high.

Further, the density of the top portion 51 may be higher than the density of the wall portion 53. Since the density of the top portion 51 and the density of the bottom portion 52 are higher than the density of the wall portion 53, the bodily fluid discharged onto the top sheet 46 is easily introduced to the top portion 51 and the bottom portion 52. When the bodily fluid is introduced into the bottom portion 52, the bodily fluid can be transferred to the auxiliary sheet 45 while introducing the bodily fluid into the side away from the body. This makes it possible to suppress the problem of the bodily fluid continuing to come into contact with the skin. Further, the bodily fluid introduced to the top portion 51 and introduced from the top portion 51 toward the auxiliary sheet 45 is introduced into the space on the back side of the bulge of the top portion 51. When the bodily fluid is drawn into the space on the back side of the top portion 51, the bodily fluid is introduced into the side away from the body, which makes it possible to suppress the problem of the bodily fluid continuing to come into contact with the skin.

The protruded and recessed structure portion 50 may also be located in the non-overlaid region R12. In the absorbent article 10, the protruded and recessed structure portion 50 is located in the rear non-overlaid region R12R, and is not located in the front non-overlaid region R12F and the side non-overlaid region R12S. In the non-overlaid region R 12, the auxiliary sheet 45 is not arranged, and the protruded and recessed structure portion 50 is not integrally formed with the auxiliary sheet 45 and the top sheet 46. Therefore, in the non-overlaid region, the drawability of bodily fluid toward the non-skin facing surface side becomes low compared with the overlaid region R11. The protruded and recessed structure portion 50 is located in the rear non-overlaid region R12R. When an excessively large amount of bodily fluid is drawn into the rear non-overlaid region R12R, there is a possibility that rear leakage may occur. However, the drawability of the bodily fluid to the non-skin facing surface side T2 of the rear non-overlaid region R12R is relatively low, and the rear leakage of the bodily fluid can be suppressed. On the other hand, the overlaid region R11 is positioned on the crotch side with respect to the rear non-overlaid region R12R, and therefore, even when a large amount of bodily fluid is drawn, rear leakage is less likely to occur. Therefore, according to the above-described configuration, when the rear non-overlaid region R12R and the overlaid region R11 are located, absorption performance can be secured while suppressing rear leakage. Further, in a modified example, the protruded and recessed structure portion 50 may also be located in the side non-overlaid region R12S. When an excessively large amount of bodily fluid is drawn into the side non-overlaid region R12S, there is a possibility that lateral leakage may occur. However, the drawability of the bodily fluid to the non-skin facing surface side T2 of the side non-overlaid region R12S is relatively low, and the lateral leakage of the bodily fluid can be suppressed. On the other hand, the overlaid region R11 is positioned on the inner side in the width direction W with respect to the side non-overlaid region R12S, and therefore, even when a large amount of bodily fluid is drawn, lateral leakage is less likely to occur. Therefore, according to the above-described configuration, when the side non-overlaid region R12S and the overlaid region R11 are located, absorption performance can be secured while suppressing lateral leakage.

The overlaid region R11 may include a first overlaid region Rill in which the protruded and recessed structure portion 50 is located, and a second overlaid region R112 in which the protruded and recessed structure portion 50 is not located. The second overlaid region R112 may be arranged on the front side with respect to the first overlaid region R111. In the first overlaid region R111 in which the protruded and recessed structure portion 50 is located, excrement can be drawn from the front surface side to the back surface side of the top sheet 46. The moisture in the drawn excrement is transferred to the non-skin facing surface side T2. In addition, components excluding moisture in the excrement remain on the top sheet 46 and are diffused along the recessed portions and the like of the protruded and recessed structure portion 50. At this time, since the second overlaid region R112 in which the protruded and recessed structure portion 50 is not located is arranged on the front side with respect to the first overlaid region R111, and the protruded and recessed structure portion 50 that diffuses soft feces are not located in the first overlaid region R111, the diffusion of excrement toward the crotch side can be suppressed. In addition, the first overlaid region R111 is configured to have a high density at the bottom portion 52, and therefore the bodily fluid is easily drawn. On the other hand, in the second overlaid region R112, the bottom portion having a high density or the like is not formed, and moisture is not easily transferred from the first overlaid region R111 toward the second overlaid region R112. Therefore, the soft feces can be prevented from being transferred to the crotch side.

The area of the first overlaid region Rill may be smaller than the area of the second overlaid region R112. Since the area of the first overlaid region R111 is relatively small, the soft feces can be locally retained and the diffusion of the soft feces can be suppressed. Therefore, the load on the skin caused by the attachment of soft feces to the skin can be reduced. The first overlaid region R111 is arranged in a region that extends toward the front side from the rear end edge (the rear end edge of the auxiliary sheet 45) of the overlaid region R11. The front end edge (the rear end edge of the second overlaid region R 112) of the first overlaid region Rill constitutes a front end edge 50F of the protruded and recessed structure portion 50, and may be arranged on the rear side with respect to the center of the absorbent article in the front-rear direction L. According to the above-described configuration, the diffusion of excrement toward the crotch side can be further suppressed. The front end edge of the second overlaid region R112 is arranged in a region that extends toward the rear side from the front end edge (the front end edge of the auxiliary sheet 45) of the overlaid region R11. According to the above-described configuration, urine can be drawn by the auxiliary sheet 45 into the center of the absorbent article in the front-rear direction L where urination frequently occurs to suppress the liquid residue on the top sheet 46.

As shown in FIG. 2, a rear end edge 50R of the protruded and recessed structure portion 50 may be positioned on the rear side with respect to the rear end edge 46R of the auxiliary sheet 45 and may be positioned on the front side with respect to a rear end edge 40aR of the absorbent core. Further, the front end edge 45F of the auxiliary sheet 45 is positioned on the front side with respect to the front end edge 50F of the protruded and recessed structure portion 50, and is positioned on the rear side with respect to the front end edge 40aF of the absorbent core 40a. Therefore, from the front end edge toward the rear side of the absorbent article, the front end edge of the absorbent article, the front end edge 46F of the top sheet 46, the front end edge 40aF of the absorbent core, the front end edge 45F of the auxiliary sheet 45, the front end edge 50F of the protruded and recessed structure portion 50, the rear end edge 45R of the auxiliary sheet 45, the rear end edge 50R of the protruded and recessed structure portion 50, the rear end edge 40aR of the absorbent core, the rear end edge of the absorbent article, and the rear end edge 46R of the top sheet 46 are arranged with spaces in the front-rear direction L.

The density of the auxiliary sheet 45 may be higher than the density of the top sheet 46. The relatively high density of the auxiliary sheet 45 makes it possible to facilitate the transfer of bodily fluid from the top sheet 46 toward the auxiliary sheet 45. It should be noted that a portion in which the density is high and a portion in which the density is low in the sheet can be determined by a difference in fiber diameter. In a case where the fiber diameter is small, it can be determined that the interfiber distance is short and the density is high. Further, the density of the core wrap 40b may be higher than the density of the auxiliary sheet 45. The relatively high density of the core wrap makes it possible to facilitate the transfer of bodily fluid from the top sheet 46 toward the auxiliary sheet 45 and the transfer of bodily fluid from the auxiliary sheet 45 toward the core wrap 40b.

The absorbent core 40a may include at least a superabsorbent polymer, and the weight of the superabsorbent polymer may be 60% or more and 100% or less with respect to the weight of the absorbent core. Since the weight of the superabsorbent polymer in the absorbent core 40a is relatively high, the absorbent core 40a easily swells when bodily fluid is absorbed, compared with a configuration in which the ratio of pulp in the absorbent core 40a is low. When the absorbent core 40a swells and the absorbent core 40a bulges toward the auxiliary sheet 45, the bottom portion of the auxiliary sheet 45 and the absorbent core 40a become closer to each other, or the absorbent core enters the recessed portion on the back side of the top portion of the auxiliary sheet 45, which makes it possible to enhance the drawability of bodily fluid toward the absorbent core. The weight of the superabsorbent polymer can be calculated based on the water retention amount of the absorbent body. The larger the weight (the higher the ratio) of the superabsorbent polymer, the larger the water retention amount of the entire absorbent body.

### (4) Method for Manufacturing Absorbent Article

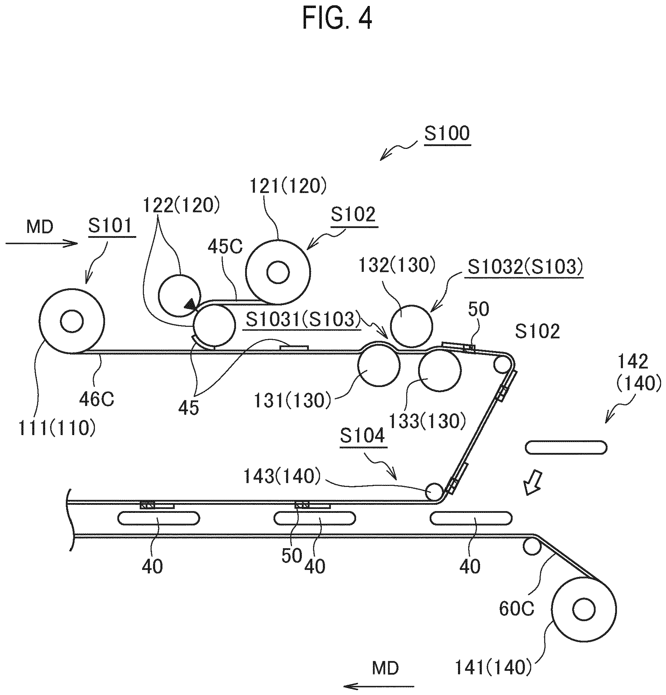

Next, a method for manufacturing an absorbent article will be described with reference to FIG. 4. FIG. 4 is a diagram schematically showing an example of a method for manufacturing an absorbent article and an apparatus for manufacturing an absorbent article. A method S 100 for manufacturing an absorbent article includes at least a top sheet transport step S 101, a laminate forming step S 102, a shaping step S103, and an overlaying step S104. The apparatus for manufacturing an absorbent article includes at least a top sheet supply mechanism 110, an auxiliary sheet supply mechanism 120, a shaping mechanism 130, and an absorbent body and back sheet supply mechanism 140.

The top sheet transport step S101 is performed by the top sheet supply mechanism 110 and transports the top sheet continuous body 46C in which the top sheet that comes into contact with the wearer is continuous. The top sheet supply mechanism 110 includes at least an unwinding roll 111 having the top sheet continuous body 46C to be processed wound thereon in a roll form and configured to unwind the top sheet continuous body 46C toward a transport direction MD.

The laminate forming step S102 is positioned on the downstream side in the transport direction MD with respect to the top sheet transport step S 101. The laminate forming step S102 is performed by the auxiliary sheet supply mechanism 120 to overlay the auxiliary sheet 45 on the top sheet continuous body 46C to form a sheet laminate. The auxiliary sheet supply mechanism 120 includes an unwinding roll 121 having an auxiliary sheet continuous body 45C in which the auxiliary sheet 45 is continuous to be processed wound thereon in a roll form and configured to unwind the auxiliary sheet continuous body 45C toward a transport direction MD, and a pair of transport rolls 122 configured to cut the auxiliary sheet continuous body 45C supplied from the unwinding roll 121 by a cutter, and overlay the cut auxiliary sheet 45 on the top sheet continuous body 46C. In the laminate forming step S102, the auxiliary sheets 45 are arranged with spaces in the transport direction MD. It should be noted that since the auxiliary sheets 45 of absorbent article 10 are arranged with spaces in the front-rear direction L, the auxiliary sheets 45 are intermittently arranged in the transport direction MD. However, in a modified example, in an aspect in which the auxiliary sheet 45 is continuous in the front-rear direction L, the auxiliary sheet continuous body may be overlaid on the top sheet continuous body.

The shaping step S103 is positioned on the downstream side in the transport direction MD with respect to the laminate forming step S102. The shaping step S103 is performed by the shaping mechanism 130 to form the protruded and recessed structure portion 50 in at least the overlaid region R11 of the sheet laminate. In the shaping step S103, the protruded and recessed structure portion 50 is formed with spaces in the front-rear direction L at position where at least a part of the protruded and recessed structure portion corresponds to the rear waist region 30. By providing the shaping step S103, an absorbent article in which the protruded and recessed structure portion 50 integrally formed with the top sheet 46 and the auxiliary sheet 45 is located in at least the rear waist region can be manufactured. In the shaping step S103, the protruded and recessed structure portions 50 may be formed with spaces in the transport direction MD. An absorbent article 10 having a region in which the protruded and recessed structure portion 50 is formed and a region in which the protruded and recessed structure portion 50 is not formed can be manufactured.

The overlaying step S 104 is positioned on the downstream side in the transport direction MD with respect to the shaping step S 103. The overlaying step S 104 is performed by the absorbent body and back sheet supply mechanism 140, and the sheet laminate subjected to the shaping process is overlaid on the absorbent body and the back sheet. The absorbent body and back sheet supply mechanism 140 includes at least an unwinding roll 141, an absorbent body supply mechanism 142, and a sheet laminate supply mechanism 143. The unwinding roll 141 has a back sheet continuous body 60C in which the back sheet 60 is continuous to be processed wound thereon in a roll form, and unwinds the back sheet continuous body 60C in the transport direction MD. The absorbent body supply mechanism 142 overlays the absorbent core 40a with a laminating drum or the like that overlays the absorbent material, covers the absorbent core 40a with the core wrap 40b to mold the absorbent body 40, and arranges the absorbent bodies 40 on the back sheet continuous body 60C with spaces in the transport direction MD. Since the sheet laminate is arranged on the absorbent body 40 after the shaping step S103, compared with a configuration in which the top sheet continuous body 46C and the auxiliary sheet 45 are separately arranged on the absorbent body, the top sheet continuous body 46C and the auxiliary sheet 45 can be integrally arranged, and problems such as sheet folding in the process of transporting the top sheet continuous body 46C and the auxiliary sheet 45 can be suppressed. Further, the sheet laminate supply mechanism 143 overlays the sheet laminate on the back sheet continuous body 60C and the absorbent body 40. Next, an absorbent article 10 can be manufactured by cutting the sheet along the outer edge of the absorbent article 10 with a cutting mechanism (not shown).

In the laminate forming step S 102, the top sheet continuous body 46C and the auxiliary sheet 45 may be joined to each other. A step of joining the top sheet continuous body 46C and the auxiliary sheet 45 may be a step before the shaping step S103. The top sheet continuous body 46C and the auxiliary sheet 45 can be integrally arranged, and problems such as sheet folding in the process of transporting the top sheet continuous body 46C and the auxiliary sheet 45 can be suppressed. It is more suitable that, in the step of joining the top sheet continuous body 46C and the auxiliary sheet 45, the top sheet continuous body 46C and the auxiliary sheet 45 may be joined by thermal welding or ultrasonic welding. According to the above-described configuration, compared with a configuration in which the top sheet continuous body and the auxiliary sheet are joined via a hot-melt adhesive, a decrease in the drawability of bodily fluid due to the adhesive can be suppressed. Therefore, the top sheet 46 and the auxiliary sheet 45 can be integrally formed while suppressing a decrease in the drawability of the bodily fluid, and the transferability of bodily fluid from the top sheet toward the auxiliary sheet 45 can be enhanced.

The shaping mechanism 130 may include at least a first shaping roll 131, a second shaping roll 132, and a third shaping roll 133. The first shaping roll 131 and the second shaping roll 132 are arranged to face each other, and a first shaping step S 1031 is performed in which a first shaping process of shaping a part of the protruded and recessed structure portion 50 is performed on the sheet laminate that passes between the rolls. Further, the second shaping roll 132 and the third shaping roll 133 are arranged to face each other, and a second shaping step S 1032 is performed in which a second shaping process of shaping the other part of the protruded and recessed structure portion 50 is performed on the sheet laminate that passes between the rolls. That is, the shaping step S 103 may include the first shaping step S1031 and the second shaping step S1032. The second shaping step S1032 is positioned on the downstream side in the transport direction MD with respect to the first shaping step S1031. Since the first shaping step S1031 and the second shaping step S 1032 are provided and the shaping processes are performed in a stepwise manner, the protruded and recessed structure portion 50 is easily formed to a detailed part compared with the step of forming the protruded and recessed structure portion at one time. Further, since the first shaping step and the second shaping step are performed using the common second shaping roll, a positional deviation between a part of the protruded and recessed structure portion 50 by the first shaping step S1031 and the other part of the protruded and recessed structure portion 50 by the second shaping step S1032 can be suppressed.

The first shaping roll 131 and the second shaping roll 132 may be formed of a combination of a disk roll and a pin roll, and similarly, the second shaping roll 132 and the third shaping roll 133 may be formed of a combination of a disk roll and a pin roll. More specifically, the disk roll includes protruding ridges arranged with fixed spaces in the roll width direction and a plurality of rows of recessed grooves located between adjacent protruding ridges. The protruding ridges and the recessed grooves are alternately located in the roll width direction, and are located in a manner of continuing in the roll circumferential direction. On the other hand, the pin roll includes a plurality of pins located on the outer circumferential surface so as to engage with the recessed grooves of the disk roll. The pins are arranged with fixed spaces in the roll width direction so as not to come into contact with the protruding ridges, and the pins are arranged substantially linearly along the outer circumferential surface in the roll circumferential direction with fixed spaces. By passing the sheet laminate between the disk roll and the pin roll, the position facing the pin and the recessed groove and the position corresponding to the protruding ridge can be stretched to form the protruded and recessed structure portion 50.

In the first shaping step S1031, the protruded and recessed structure portion 50 may be shaped with spaces in the first direction which is one of the front-rear direction L and the width direction W, and in the second shaping step S1032, the protruded and recessed structure portion 50 may be shaped with spaces in the second direction which is the other of the front-rear direction L and the width direction W. For example, the protruded and recessed structure portion 50 formed by the first shaping step S1031 may have the first top portion 511, the first bottom portion 521, and the first wall portion 531, and the protruded and recessed structure portion 50 formed by the second shaping step S1032 may have the second top portion 512, the second bottom portion 522, and the second wall portion 532. When the protruded and recessed structure portion 50 with spaces in the first direction and the protruded and recessed structure portion 50 with spaces in the second direction are shaped at one time, the material is less likely to stretch at the time of the shaping process. More specifically, when the protruded and recessed structure portion 50 is formed with spaces in the first direction, the material is shaped while extending in the first direction. At the same time, when the protruded and recessed structure portion 50 is formed with spaces in the second direction, the material is less likely to extend in the second direction, and there is a risk that the material may be broken or the protruded and recessed structure portion 50 cannot be appropriately molded. However, since the protruded and recessed structure portion 50 with spaces in the first direction and the protruded and recessed structure portion 50 with spaces in the second direction can be formed in a stepwise manner, the protruded and recessed structure portion 50 can be appropriately molded while preventing damage to the material.

Further, in the shaping step S 103, the protruded and recessed structure portion 50 may be formed in a part of the sheet laminate in a crossing direction CD that is orthogonal to the transport direction MD. By forming the protruded and recessed structure portion 50 in a part in the crossing direction CD instead of in the entire region of the sheet laminate in the crossing direction CD, a portion to be stretched and a portion not to be stretched can be located at the time of the shaping process, which makes it possible to stabilize the position of the stretched portion and makes it easier to form the protruded and recessed structure portion 50 in an intended position and shape. Further, when the stretched portion and the non-stretched portion are located at the time of the shaping process, the material can be prevented from being broken due to the stretching of the material at the time of the shaping process.

As described above, although the contents of the present invention were disclosed through the embodiments of the present invention, it should not be understood that the description or drawing constituting any part of this disclosure limits the present invention. From this disclosure, various alternative embodiments, examples and operational techniques will be apparent to those skilled in the art. Therefore, the technical scope of the present invention is defined only by the appended claims.

For example, the absorbent body is not limited to the configuration described in the above embodiment. The absorbent body may have a low-basis-weight portion that extends in the front-rear direction L and has a lower basis weight of the absorbent material than the surroundings. The low-basis-weight portion may straddle the overlaid region R 11 and the front non-overlaid region R12F.

### [Industrial applicability]

According to the present aspect, an absorbent article capable of enhancing the transferability of a bodily fluid to the non-skin surface on the top sheet having a protruded and recessed structure portion can be provided.

### REFERENCE SIGNS LIST

10: disposable diaper, 20: front waist region, 25: crotch region, 30: rear waist region, 40: absorbent body, 45: auxiliary sheet, 46: top sheet, 46C: top sheet continuous body, 50: protruded and recessed structure portion, 51: top portion, 52: bottom portion, 53: wall portion, 60: back sheet, 110: top sheet supply mechanism, 120: auxiliary sheet supply mechanism, 130: shaping mechanism, 140: absorbent body and back sheet supply mechanism, R11: overlaid region, R111: first overlaid region, R112: second overlaid region, R12: non-overlaid region, S101: top sheet transport step, S102: laminate forming step, S103: shaping step, S1031: first shaping step, S1032: second shaping step, S104: overlaying step, L: front-rear direction, T: thickness direction, T1: skin facing surface side, T2: non-skin facing surface side, W: width direction

## Claims

1. A method for manufacturing an absorbent article (10) having a front waist region (20), a rear waist region (30), and a crotch region (25) that is positioned between the front waist region (20) and the rear waist region (30), and having a front-rear direction (L) that is directed from the front waist region (20) toward the rear waist region (30), and a width direction (W) that is orthogonal to the front-rear direction (L), the method comprising:
a top sheet transport step (S 101) of transporting a top sheet continuous body (46C) in which a top sheet (46) that comes into contact with a wearer is continuous;
a laminate forming step (S 102) of overlaying an auxiliary sheet (45) that comes into contact with a non-skin surface of the top sheet (46) on the top sheet continuous body (46C) to form a sheet laminate; and
a shaping step (S 103) of forming a protruded and recessed structure portion (50) having a top portion (51) that protrudes toward a skin facing surface side (T1), a bottom portion (52) that protrudes toward a non-skin facing surface side (T2), and a wall portion (53) that connects the top portion (51) and the bottom portion (52) in an overlaid region (R11) in which the top sheet continuous body (46C) and the auxiliary sheet (45) overlap each other in the sheet laminate, wherein
in the shaping step (S 103), the protruded and recessed structure portion (50) is formed with spaces in the front-rear direction (L) at a position where at least a part of the protruded and recessed structure portion (50) corresponds to the rear waist region (30).

2. The method for manufacturing an absorbent article (10) according to claim 1, wherein
in the laminate forming step (S 102), the top sheet continuous body (46C) and the auxiliary sheet (45) are joined by thermal welding or ultrasonic welding.

3. The method for manufacturing an absorbent article (10) according to claim 1 or 2, wherein
the shaping step (S103) includes a first shaping step (S1031) of allowing the sheet laminate to pass between a first shaping roll (131) and a second shaping roll (132) that face each other to shape a part of the protruded and recessed structure portion (50), and a second shaping step (S 1032) of allowing the sheet laminate to pass between the second shaping roll (132) and a third shaping roll (133) that face each other to shape the other part of the protruded and recessed structure portion (50) after the first shaping step (S1031).

4. The method for manufacturing an absorbent article (10) according to claim 3, wherein
in the first shaping step (S 1031), the protruded and recessed structure portion (50) is shaped with spaces in a first direction that is one of the front-rear direction (L) and the width direction (W), and
in the second shaping step (S 1032), the protruded and recessed structure portion (50) is shaped with spaces in a second direction that is the other of the front-rear direction (L) and the width direction (W).

## Patentansprüche

1. Ein Verfahren zur Herstellung eines saugfähigen Artikels (10), der einen vorderen Taillenbereich (20), einen hinteren Taillenbereich (30) und einen zwischen dem vorderen Taillenbereich (20) und dem hinteren Taillenbereich (30) positionierten Schrittbereich (25) aufweist und der eine von vorne nach hinten verlaufende Richtung (L), die von dem vorderen Taillenbereich (20) hin zu dem hinteren Taillenbereich (30) gerichtet ist, und eine Breitenrichtung (W), die orthogonal zu der von vorne nach hinten verlaufenden Richtung (L) liegt, aufweist, wobei das Verfahren Folgendes beinhaltet:
einen Deckschicht-Transportschritt (S101) zum Transportieren eines kontinuierlichen Deckschichtkörpers (46C), wobei eine Deckschicht (46), die mit einem Träger in Kontakt kommt, kontinuierlich ist;
einen Schichtstoffbildungsschritt (S102) zum Überlagern einer Hilfsschicht (45), die mit einer Nicht-Hautoberfläche der Deckschicht (46) auf dem kontinuierlichen Deckschichtkörper (46C) in Kontakt kommt, um einen flächigen Schichtstoff zu bilden; und
einen Formungsschritt (S103) zum Bilden eines Strukturabschnitts mit Vorsprüngen und Vertiefungen (50), der Folgendes aufweist: einen oberen Abschnitt (51), der zu einer zur Haut gerichteten Oberflächenseite (T1) vorspringt, einen unteren Abschnitt (52), der zu einer nicht zur Haut gerichteten Oberflächenseite (T2) vorspringt, und einen Wandabschnitt (53), der den oberen Abschnitt (51) und den unteren Abschnitt (52) in einem überlagerten Bereich (R11) verbindet, in dem der kontinuierliche Deckschichtkörper (46C) und die Hilfsschicht (45) einander in dem flächigen Schichtstoff überlappen, wobei
in dem Formungsschritt (S103) der Strukturabschnitt mit Vorsprüngen und Vertiefungen (50) mit Zwischenräumen in der von vorne nach hinten verlaufenden Richtung (L) an einer Position gebildet wird, an der mindestens ein Teil des Strukturabschnitts mit Vorsprüngen und Vertiefungen (50) dem hinteren Taillenbereich (30) entspricht.

2. Verfahren zur Herstellung eines saugfähigen Artikels (10) gemäß Anspruch 1, wobei
in dem Schichtstoffbildungsschritt (S 102) der kontinuierliche Deckschichtkörper (46C) und die Hilfsschicht (45) durch Wärmeschweißen oder Ultraschallschweißen miteinander verbunden werden.

3. Verfahren zur Herstellung eines saugfähigen Artikels (10) gemäß Anspruch 1 oder 2, wobei
der Formungsschritt (S103) Folgendes umfasst: einen ersten Formungsschritt (S 1031) zum Ermöglichen, dass der flächige Schichtstoff zwischen einer ersten Formwalze (131) und einer zweiten Formwalze (132), die einander gegenüberliegen, hindurchtritt, um einen Teil des Strukturabschnitts mit Vorsprüngen und Vertiefungen (50) zu formen, und einen zweiten Formungsschritt (S1032) zum Ermöglichen, dass der flächige Schichtstoff zwischen der zweiten Formwalze (132) und einer dritten Formwalze (133), die einander gegenüberliegen, hindurchtritt, um den anderen Teil des Strukturabschnitts mit Vorsprüngen und Vertiefungen (50) nach dem ersten Formungsschritt (S1031) zu formen.

4. Verfahren zur Herstellung eines saugfähigen Artikels (10) gemäß Anspruch 3, wobei
in dem ersten Formungsschritt (S 1031) der Strukturabschnitt mit Vorsprüngen und Vertiefungen (50) mit Zwischenräumen in einer ersten Richtung geformt wird, die eine von der von vorne nach hinten verlaufenden Richtung (L) und der Breitenrichtung (W) ist, und
in dem zweiten Formungsschritt (S 1032) der Strukturabschnitt mit Vorsprüngen und Vertiefungen (50) mit Zwischenräumen in einer zweiten Richtung geformt wird, die die andere von der von vorne nach hinten verlaufenden Richtung (L) und der Breitenrichtung (W) ist.

## Revendications

1. Procédé de fabrication d'un article absorbant (10) ayant une région avant au niveau de la taille (20), une région arrière au niveau de la taille (30), et une région au niveau de l'entrejambe (25) qui est positionnée entre la région avant au niveau de la taille (20) et la région arrière au niveau de la taille (30), et ayant une direction allant dans le sens avant-arrière (L) qui est dirigée depuis la région avant au niveau de la taille (20) vers la région arrière au niveau de la taille (30), et une direction allant dans le sens de la largeur (W) qui est orthogonale par rapport à la direction allant dans le sens avant-arrière (L), le procédé comportant :
une étape de transport de feuille supérieure (S101) consistant à transporter un corps continu de feuille supérieure (46C) dans lequel une feuille supérieure (46) qui entre en contact avec un utilisateur est continue ;
une étape de formation de stratifié (S102) consistant à recouvrir une feuille auxiliaire (45) qui entre en contact avec une surface non orientée vers la peau de la feuille supérieure (46) sur le corps continu de feuille supérieure (46C) pour former un stratifié de feuilles ; et
une étape de mise en forme (S103) consistant à former une partie de structure faisant saillie et en retrait (50) ayant une partie supérieure (51) qui fait saillie vers un côté de surface orienté vers la peau (T1), une partie inférieure (52) qui fait saillie vers un côté de surface non orienté vers la peau (T2), et une partie de paroi (53) qui relie la partie supérieure (51) et la partie inférieure (52) dans une région superposée (R11) dans laquelle le corps continu de feuille supérieure (46C) et la feuille auxiliaire (45) se chevauchent l'un l'autre dans le stratifié de feuilles, dans lequel
au cours de l'étape de mise en forme (S103), la partie de structure faisant saillie et en retrait (50) est formée avec des espaces dans la direction allant dans le sens avant-arrière (L) au niveau d'une position où au moins une partie de la partie de structure faisant saillie et en retrait (50) correspond à la région arrière au niveau de la taille (30).

2. Procédé de fabrication d'un article absorbant (10) selon la revendication 1, dans lequel
au cours de l'étape de formation de stratifié (S102), le corps continu de feuille supérieure (46C) et la feuille auxiliaire (45) sont reliés par soudage thermique ou par soudage ultrasonique.

3. Procédé de fabrication d'un article absorbant (10) selon la revendication 1 ou la revendication 2, dans lequel
l'étape de mise en forme (S103) comprend une première étape de mise en forme (S1031) consistant à permettre au stratifié de feuilles de passer entre un premier rouleau de mise en forme (131) et un deuxième rouleau de mise en forme (132) qui sont orientés l'un vers l'autre pour effectuer une mise en forme d'une partie de la partie de structure faisant saillie et en retrait (50), et une deuxième étape de mise en forme (S1032) consistant à permettre au stratifié de feuilles de passer entre le deuxième rouleau de mise en forme (132) et un troisième rouleau de mise en forme (133) qui sont orientés l'un vers l'autre pour effectuer une mise en forme de l'autre partie de la partie de structure faisant saillie et en retrait (50) après la première étape de mise en forme (S1031).

4. Procédé de fabrication d'un article absorbant (10) selon la revendication 3, dans lequel
au cours de la première étape de mise en forme (S1031), la partie de structure faisant saillie et en retrait (50) est mise en forme avec des espaces dans une première direction qui est l'une parmi la direction allant dans le sens avant-arrière (L), et la direction allant dans le sens de la largeur (W), et
au cours de la deuxième étape de mise en forme (S1032), la partie de structure faisant saillie et en retrait (50) est mise en forme avec des espaces dans une deuxième direction qui est l'autre parmi la direction allant dans le sens avant-arrière (L) et la direction allant dans le sens de la largeur (W).
